# EUROPEAN PATENT SPECIFICATION

(11) **EP 2 710 976 B1**
(45) Date of publication and mention of the grant of the patent: **15.10.2014**
(21) Application number: 13183499.6
(22) Date of filing: 09.09.2013
(51) Int. Cl.: A61B 19/08

(54) **Medical oral treatment face mask and method for using the same**
Gesichtsmaske zur medizinischen oralen Behandlung und Verfahren zur Verwendung davon
Masque facial de traitement buccal médical et son procédé d'utilisation

(30) Priority: 19.09.2012 TW 101134377
(43) Date of publication of application: 26.03.2014
(73) Proprietor: Center Healthcare Technology Co., Ltd., 110 Taipei City (TW)
(72) Inventor: Cheng, Yung-Chu, 110 Taipei City (TW); Cheng, Chien-Feng, 110 Taipei City (TW); Cheng, Yu-Liang, 110 Taipei City (TW)
(74) Representative: Urner, Peter

(56) References cited:
- US-A- 3 478 432
- US-A- 4 626 211
- US-A- 4 969 473
- US-A- 6 079 980
- US-B1- 6 185 740

## Description

### Background of the Invention

### 1. Technical Field

The invention relates to face masks, particularly to medical oral treatment face masks.

### 2. Related Art

Oral treatment is a recognized international specialty in dentistry. It includes the diagnosis, surgical and related treatment of diseases, injuries and defects involving both the functional and esthetic aspects of the hard and soft tissues of the head, mouth, teeth, gums, jaws and neck.

Oral treatment as a specialty is usually defined as a dental specialty. When a patient is being treated, he or she must open his or her mouth to be checked and operated by a dentist. A liquid suction tube also has to be put in the patient's mouth to drain out saliva, blood and swarf.

However, water injected from a dental water jet or the patient's saliva can easily splash or flow out. This may make the patient feel uncomfortable and may stain the patient's clothing.

US 6,079,980 is concerned with a dental patient face mask that covers the patient's entire face. The mask consists of a masking sheet that concludes a molded nose mask, a sight aperture and a mouth cut-out. The masking sheet is of a three-ply material having a waterproof inner layer sandwiched between two absorbent outer layers. This document is the closest prior art to the subject-matter of the invention.

US 4,626,211 A discloses a dental shield consisting of a panel composed of a two-ply sheet of paper having a plastic sheet adhered to its back. A mouth opening is provided through the panel which aligns with a similar mouth opening through a cotton mouth guard. The mouth guard is of cotton for absorbing purposes and is adhered to the rear side of the panel for engagement with the patient's face.

US 4,969,473 discloses a dental patient face and neck shield. This shield includes a mouthpiece with a corresponding opening. The shield includes one panel of an air permeable, absorbent material.

US 6,185,740 B1 discloses a face mask made of tissue and having pockets to accommodate shaping stays that can be manually conformed to the facial features of the user.

### Summary of the Invention

An object of the invention is to provide a medical oral treatment face mask, which is provided with an opening and a water-absorbent layer to absorb liquid from a patient's mouth. This can avoid a patient feeling uncomfortable or prevent a patient's clothing being stained.

To accomplish the above object, the medical oral treatment face mask of the invention includes a mask body and a band set. The mask body includes a water-repellent layer, an isolation layer and a water-absorbent layer. The isolation layer has an opening associated with the water-repellent layer. The water-absorbent layer is arranged between the water-repellent layer and the isolation layer. The band set is fixed on two sides of the mask body.

To accomplish the above object, the method for using a medical oral treatment face mask includes the steps of:
a) wearing the mask body on a person's face; and
b) expanding the opening to expose the mouth of the wearer.

The invention also has advantages as follows:
1. Liquid splashed from a patient's mouth can be absorbed by the water-absorbent layer. This can prevent the patient from feeling uncomfortable and keep clothing clean.
2. The mask body can prevent medical apparatus, the medical care personnel or other matters from touching the patient's face and can prevent drugs from being accidently absorbed by the patient. This can increase the safety during oral treatment.
3. The water-absorbent layer is extending from the bottom edge to half the area of the mask body approximately. In other words, all the upper half of the mask body is highly breathable. This makes it possible for the patient to smoothly and comfortably breathe with the mask on.
4. Because the isolation layer can be a breathable filter fabric or a meshed fabric which is not water absorbent, the water-absorbent layer can be isolated with the isolation layer. As a result, the water-absorbent layer can be prevented from direct contact with the patient's face. This can keep the patient dry and clean.
5. Because the water-repellent layer, isolation layer and water-absorbent layer jointly form the pleats, the mask of the invention can fit the patient's face.
6. The water-absorbent layer is disposed extending from the bottom edge of the mask body to three-fifths of the total area approximately or it can be formed with an aperture over the opening. Thus, the water-absorbent layer can surround the patient's mouth to enhance water absorption.
7. The mask further includes a filter layer. The filter layer can purify air breathed by the patient to prevent the patient from infection.
8. The mask body is formed with a bag extending and folded from the bottom edge, and the water-absorbent layer is replaceably received in the bag. Thus, the water-absorbent layer can be easily replaced.

### Brief Description of the Drawings

FIG. 1 is a schematic view of the invention;
FIG. 2 is a plan view of the invention;
FIG. 3 is a sectional view of the first embodiment of the invention;
FIG. 4 is a sectional view of the second embodiment of the invention;
FIG. 5 is a plan view of the third embodiment of the invention;
FIG. 6 is a flowchart of the method for using the mask of the invention;
FIG. 7 is a schematic view of the mask in use;
FIG. 8 is another schematic view of the mask in use;
FIG. 9 is a schematic view of the fourth embodiment of the invention;
FIG. 10 is a schematic view of the fifth embodiment of the invention;
FIG. 11 is a schematic view of the sixth embodiment of the invention;
FIG. 12 is a sectional view of the seventh embodiment of the invention;
FIG. 13 is a sectional view of the eighth embodiment of the invention;
FIG. 14 is a schematic view of an exemplary; and
FIG. 15 is a schematic view of the ninth embodiment of the invention.

### Detailed Description of the Invention

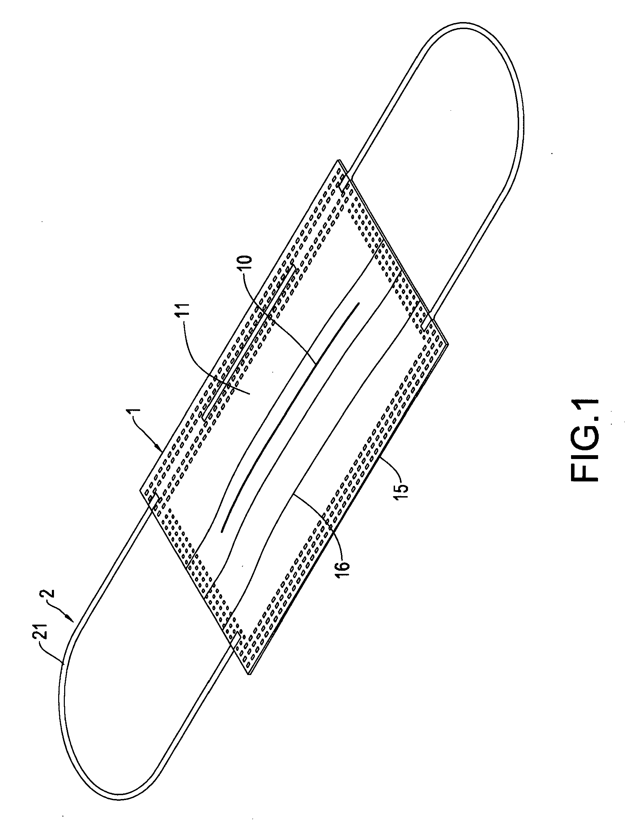

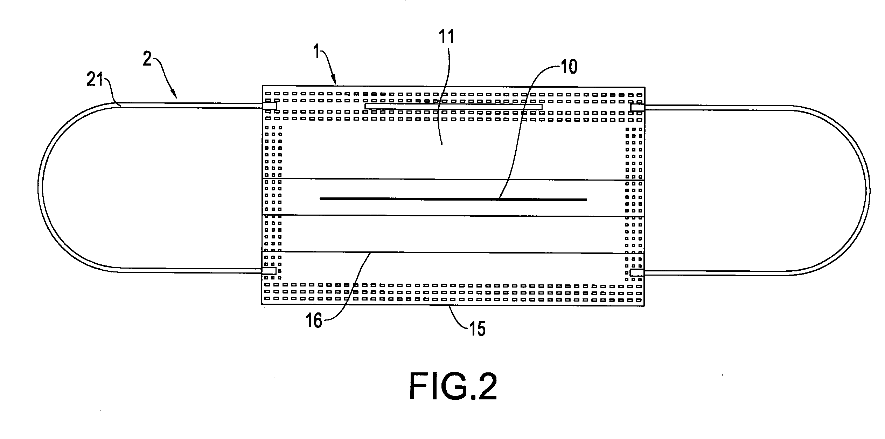

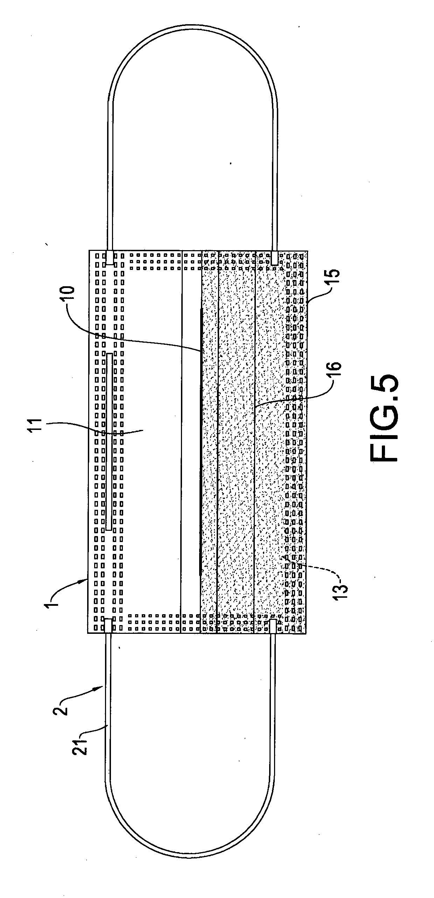

Please refer to FIGS. 1-5. The medical oral treatment face mask of the invention comprises a mask body 1 and a band set 2.

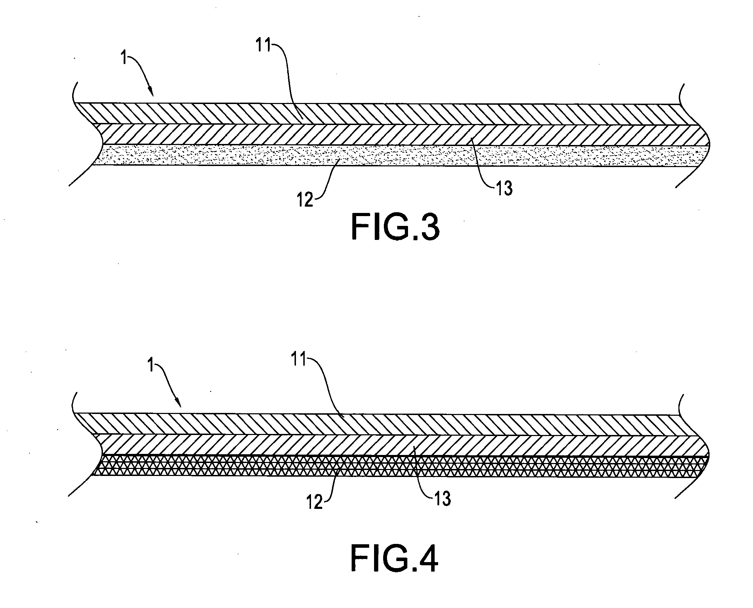

An opening10 is formed at the center of the mask body 1. The mask body 1 comprises a water-repellent layer 11, an isolation layer 12 and a water-absorbent layer 13. The water-repellent layer 11 is a breathable water-repellent fabric. The isolation layer 12 may be a breathable filter fabric as shown in FIG. 3 or a meshed fabric which is not water absorbent, as shown in FIG. 4. The water-absorbent layer 13 is a water-absorbent fabric.

The water-absorbent layer 13 is sandwiched between the water-repellent layer 11 and the isolation layer 12. The mask body 1 has a bottom edge 15 and the water-absorbent layer 13 is situated between the bottom edge 15 and opening 10.

The water-repellent layer 11 and the isolation layer 12 jointly form the opening 10. The water-repellent layer 11, isolation layer 12 and water-absorbent layer 13 jointly form pleats 16.

The band set 2 is fixed to two sides of the mask body 1 and includes two bands 21. Two ends of each band 21 is fixed to the mask body 1. The bands 21 may be elastics or ties.

Thus, liquid splashed from a patient's mouth can be absorbed by the water-absorbent layer 13. This can prevent the patient from feeling uncomfortable and keep clothing clean.

The mask body 1 can prevent medical apparatus, the medical care personnel or other matters from touching the patient's face and can prevent drugs from being accidently absorbed by the patient. This can increase the safety during oral treatment.

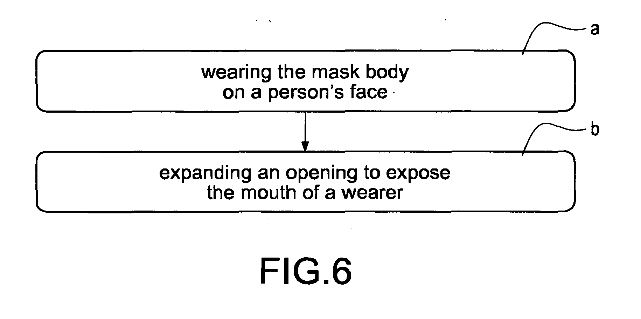

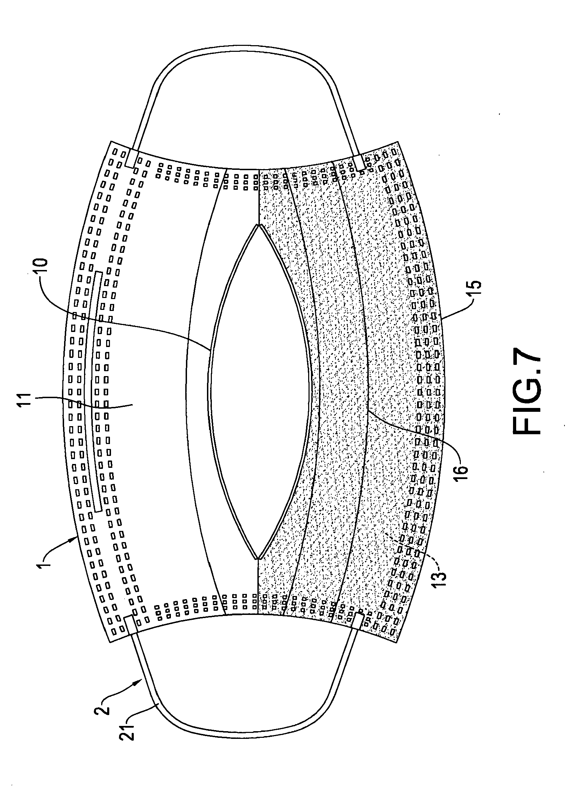

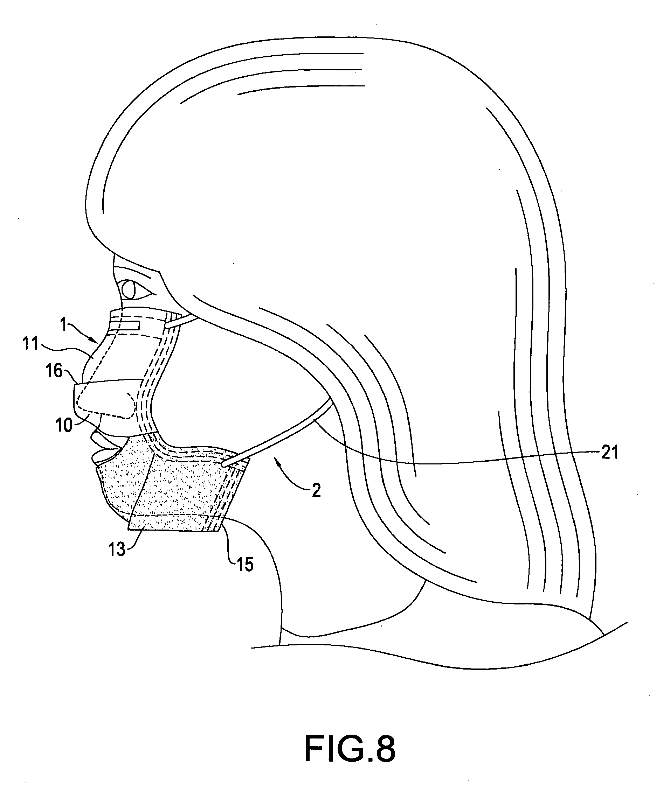

Please refer to FIGS. 6-8, which show the method for using the face mask of the invention. The method includes two steps: a) wearing the mask body 1 on a person's face; and b) expending the opening 10 to expose the mouth of the wearer (person). As a result, a dentist can treat the patient's mouth through the opening 10 and the water-absorbent layer 13 will absorb the liquid flowing or splashing out.

Additionally, the water-absorbent layer 13 is less breathable and extending from the bottom edge 15 to half the area of the mask body 1 approximately. In other words, all the upper half of the mask body 1 is highly breathable. This makes it possible for the patient to smoothly and comfortably breathe with the mask on.

Because the isolation layer 12 can be a breathable filter fabric or a meshed fabric which is not water absorbent, and the water-absorbent layer 13 is a water-absorbent fabric, the water-absorbent layer 13 can be isolated with the isolation layer 12. As a result, the water-absorbent layer 13 can be prevented from direct contact with the patient's face. This can keep the patient dry and clean.

Because the water-repellent layer 11, isolation layer 12 and water-absorbent layer 13 jointly form the pleats 16, the mask of the invention can fit the patient's face.

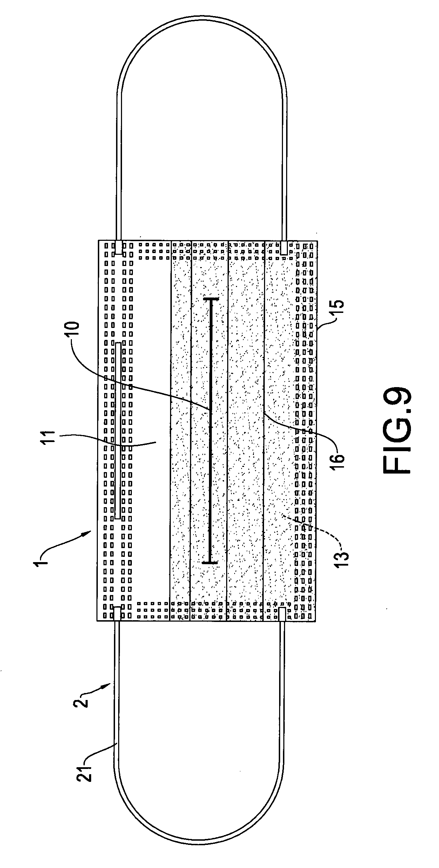

FIG. 9 shows a fourth embodiment of the invention. The water-absorbent layer 13 is disposed extending from the bottom edge 15 of the mask body 1 to three-fifths of the total area approximately. Thus, the water-absorbent layer 13 can surround the patient's mouth to enhance water absorption.

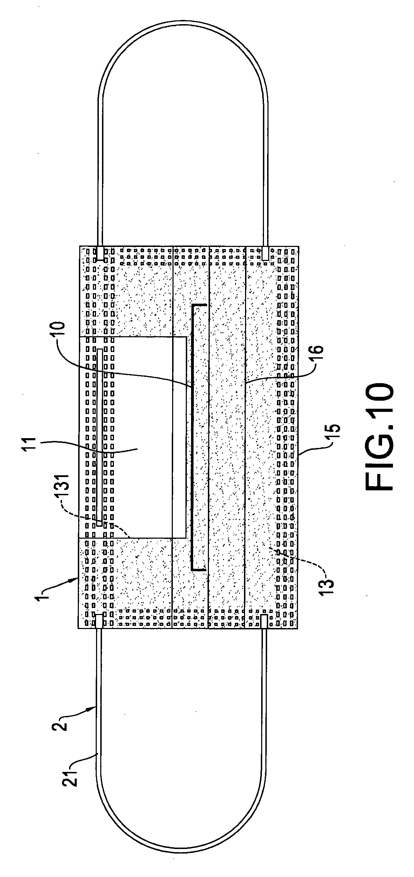

FIG. 10 shows a fifth embodiment of the invention. The water-absorbent layer 13 is formed with an aperture 131 over the opening 10. The aperture 131 corresponds to a patient's nose. Thus, the portion of the mask body 1 which corresponds to the patient's nose is highly breathable to keep breathing smooth, and the water-absorbent layer 13 can surround the patient's mouth to enhance water absorption.

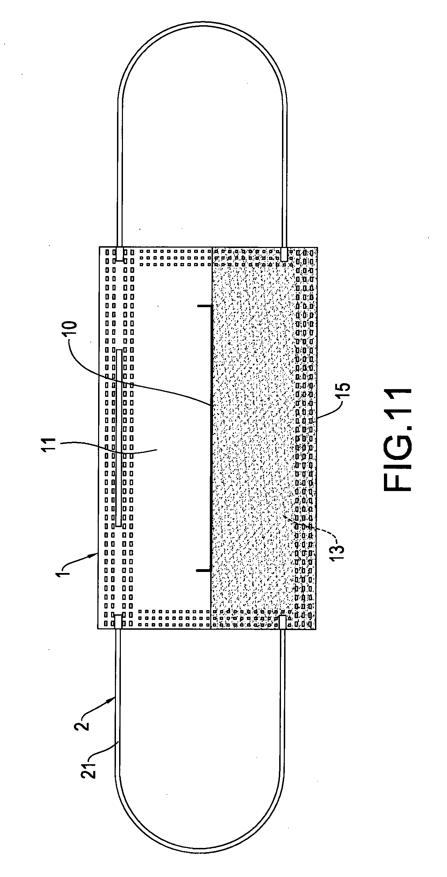

FIG. 11 shows a sixth embodiment of the invention. The water-repellent layer 11, isolation layer 12 and water-absorbent layer 13 can be formed pleats 16 of any shapes and folded as a planar. Thus, a dentist can treat the patient's mouth through the opening 10 and the water-absorbent layer 13 will absorb the liquid flowing or splashing out.

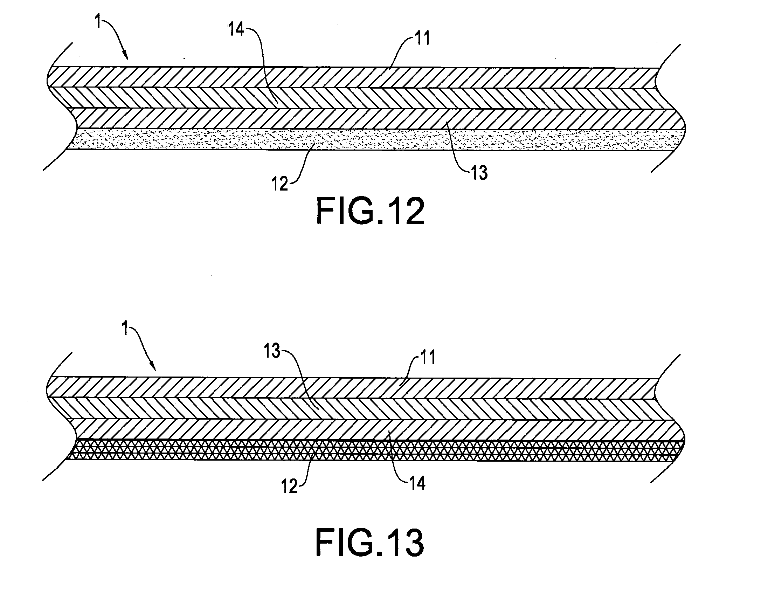

FIG. 12 and 13 show a seventh embodiment and an eighth embodiment of the invention. The mask further includes a filter layer 14. The filter layer 14 is disposed between the water-repellent layer 11 and the water-absorbent layer 13 or between the isolation layer 12 and the water-absorbent layer 13. The filter layer 14 is a breathable fabric. The water-repellent layer 11, the isolation layer 12, the water-absorbent layer 13 and the filter layer 14 jointly form pleats 16. The filter layer 14 can purify air breathed by the patient to prevent the patient from infection.

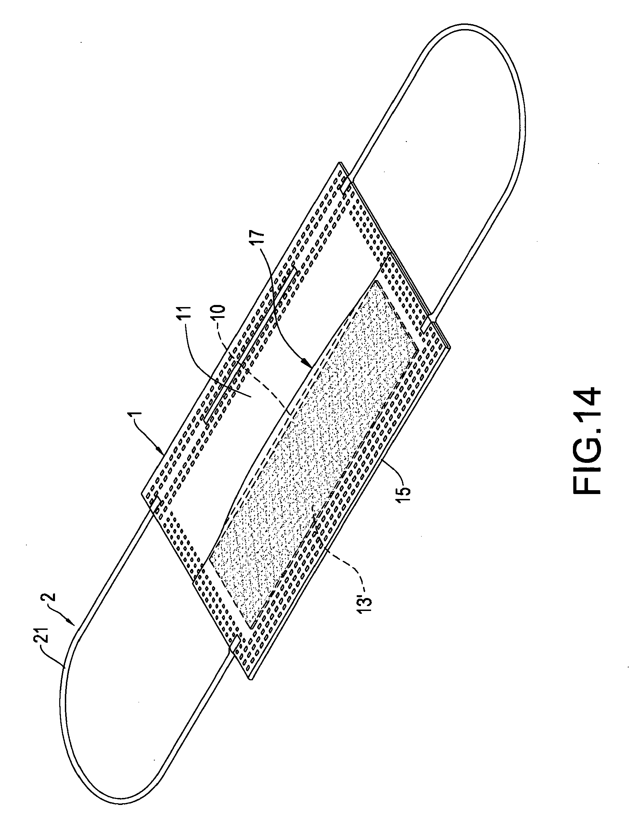

FIG. 14 shows an exemplary face mask. This face mask includes a mask body 1 and a water-absorbent layer 13'. The mask body 1 is formed with an opening 10. The water-absorbent layer 13' is a water-absorbent fabric around the opening 10.

The mask body 1 is formed with a bag 17 extending and folded from the bottom edge 15, and the water-absorbent layer 13' is replaceably received in the bag. Thus, the water-absorbent layer 13' can be easily replaced.

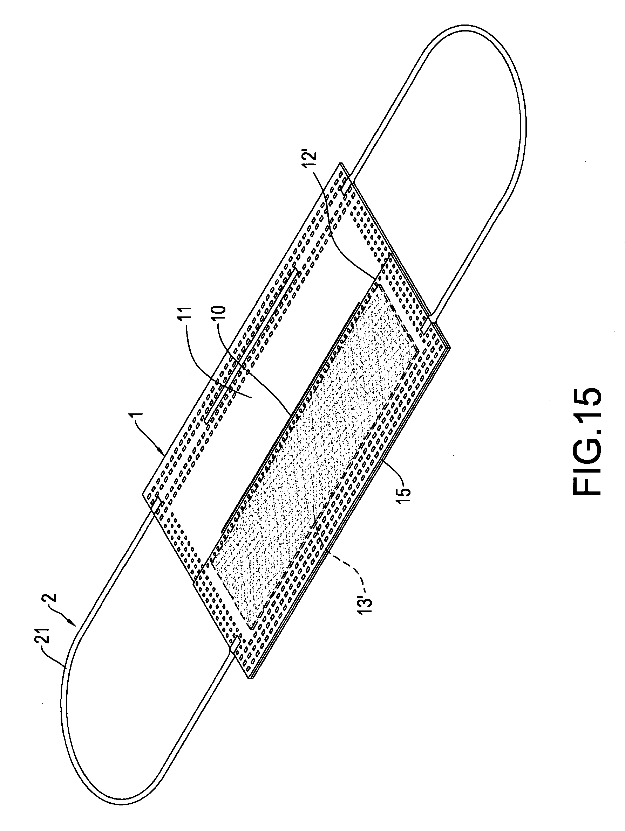

FIG. 15 shows a ninth embodiment of the invention. This embodiment includes a mask body 1 and a water-absorbent layer 13'. The mask body 1 is formed with an opening 10. The water-absorbent layer 13' is a water-absorbent fabric around the opening 10.

This embodiment further includes an isolation layer 12', which may be a breathable or meshed fabric. The isolation layer 12' and the water-absorbent layer 13' are jointly situated between the bottom edge 15 and the opening 10.

While the foregoing is directed to preferred embodiments of the present invention, other and further embodiments of the invention may be devised without departing from the basic scope thereof. As such, the appropriate scope of the invention is to be determined according to the claims.

## Claims

1. A medical oral treatment face mask comprising:
a mask body (1) comprising:
a water-repellent layer (11);
an isolation layer (12) which along with the water-repellent layer (11) has a corresponding opening (10); and
a water-absorbent layer (13) arranged between the water-repellent layer (11) and the isolation layer (12); and
a band set (2), fixed on two sides of the mask body (1).

2. The medical oral treatment face mask of claim 1, wherein the opening (10) is located at the center of the mask body (1).

3. The medical oral treatment face mask of claim 1, wherein the opening (10) is jointly formed on the water-repellent layer (11), the isolation layer (12) and the water-absorbent layer (13), and the water-absorbent layer (13) is formed with an aperture (131) over the opening (10).

4. The medical oral treatment face mask of claim 1, wherein the mask body (1) has a bottom edge (15), and the water-absorbent layer (13) is situated between the bottom edge (15) and the opening (10).

5. The medical oral treatment face mask of claim 1, further comprising a filter layer (14).

6. The medical oral treatment face mask of claim 1, wherein the band set (2) includes two bands (21) separately fixed on two sides of the mask body (1).

7. A method for using a medical oral treatment face mask of claim 1, comprising:
a) wearing the mask body (1) on a person's face; and
b) expanding an opening (10) to expose the mouth of a wearer.

## Patentansprüche

1. Gesichtsmaske für die medizinische orale Behandlung, die umfasst:
einen Maskenkörper (1), der umfasst:
eine wasserabweisende Schicht (11);
eine Isolierschicht (12), die gemeinsam mit der wasserabweisenden Schicht (11) eine entsprechende Öffnung (10) besitzt; und
eine wasserabsorbierende Schicht (13), die zwischen der wasserabweisenden Schicht (11) und der Isolierschicht (12) angeordnet ist; und
eine Bandgruppe (2), die an zwei Seiten des Maskenkörpers (1) befestigt ist.

2. Gesichtsmaske für die medizinische orale Behandlung (1), wobei sich die Öffnung (10) in der Mitte des Maskenkörpers (1) befindet.

3. Gesichtsmaske für die medizinische orale Behandlung nach Anspruch 1, wobei die Öffnung (10) zusammen auf der wasserabweisenden Schicht (11), der Isolierschicht (12) und der wasserabsorbierenden Schicht (13) gebildet ist und die wasserabsorbierende Schicht (13) mit einem Durchlass (131) über der Öffnung (10) gebildet ist.

4. Gesichtsmaske für die medizinische orale Behandlung nach Anspruch 1, wobei der Maskenkörper (1) eine untere Kante (15) besitzt und sich die wasserabsorbierende Schicht (13) zwischen der unteren Kante (15) und der Öffnung (10) befindet.

5. Gesichtsmaske für die medizinische orale Behandlung nach Anspruch 1, die ferner eine Filterschicht (14) umfasst.

6. Gesichtsmaske für die medizinische orale Behandlung nach Anspruch 1, wobei die Bandgruppe (2) zwei Bänder (21) enthält, die getrennt auf zwei Seiten des Maskenkörpers (1) befestigt sind.

7. Verfahren für die Verwendung einer Gesichtsmaske für eine medizinische orale Behandlung nach Anspruch 1, das umfasst:
a) Tragen des Maskenkörpers (1) auf dem Gesicht einer Person; und
b) Erweitern einer Öffnung (10), um den Mund des Trägers freizulegen.

## Revendications

1. Masque facial de traitement buccal médical comprenant :
un corps de masque (1) comprenant :
une couche hydrofuge (11) ;
une couche isolante (12) qui conjointement à la couche hydrofuge (11) a une ouverture correspondante (10) ; et
une couche absorbant l'eau (13) disposée entre la couche hydrofuge (11) et la couche isolante (12) ; et
un jeu de bandes (2), fixé sur les deux côtés du corps de masque (1).

2. Masque facial de traitement buccal médical selon la revendication 1, dans lequel l'ouverture (10) se trouve au centre du corps de masque (1).

3. Masque facial de traitement buccal médical selon la revendication 1, dans lequel l'ouverture (10) est formée conjointement sur la couche hydrofuge (11), la couche isolante (12) et la couche absorbant l'eau (13), et la couche absorbant l'eau (13) est formée avec un découpage (131) au-dessus de l'ouverture (10).

4. Masque facial de traitement buccal médical selon la revendication 1, dans lequel le corps de masque (1) a un bord inférieur (15), et la couche absorbant l'eau (13) se trouve entre le bord inférieur (15) et l'ouverture (10).

5. Masque facial de traitement buccal médical selon la revendication 1, comprenant en outre une couche filtrante (14).

6. Masque facial de traitement buccal médical selon la revendication 1, dans lequel le jeu de bandes (2) comprend deux bandes (21) séparément fixées sur les deux côtés du corps de masque (1).

7. Procédé d'utilisation d'un masque facial de traitement buccal médical selon la revendication 1, comprenant :
a) le port du corps de masque (1) sur le visage d'un individu ; et
b) l'agrandissement de l'ouverture (10) pour exposer la bouche d'un utilisateur.
